Europäisches Patentamt

European Patent Office

Office européen des brevets

(11) Publication number: **0 204 727**
**B1**

# EUROPEAN PATENT SPECIFICATION

(45) Date of publication of the patent specification:
**08.06.88**

(51) Int. Cl.⁴: **A 61 M 5/20,** A 61 M 1/00

(21) Application number: **85905608.7**

(22) Date of filling: **31.10.85**

(86) International application number:
**PCT/DK 85/00103**

(87) International publication number:
**WO 86/02562 (09.05.86 Gazette 86/10)**

(54) **A DRIVE MECHANISM FOR AXIAL MOVEMENT OF A ROD, IN PARTICULAR AN INFUSION PUMP.**

(30) Priority: **01.11.84  DK 5198/84**

(43) Date of publication of application:
**17.12.86 Bulletin 86/51**

(45) Publication of the grant of the patent:
**08.06.88 Bulletin 88/23**

(84) Designated Contracting States:
**AT BE CH DE FR GB IT LI LU NL SE**

(56) References cited:
**GB-A-2 077 599**
**US-A-3 395 704**

(73) Proprietor: **Nordisk Gentofte, Niels Steensensvej 1, DK- 2820 Gentofte (DK)**

(72) Inventor: **TULLIN, Flemming, Hedeparken 1, DK-2750 Ballerup (DK)**

(74) Representative: **Selting, Günther, Patentanwälte Von Kreisler- Schönwald- Fues- Keller Selting-Werner Deichmannhaus am Hauptbahnhof, D-5000 Köln 1 (DE)**

EP 0 204 727 B1

## Description

The invention concerns a drive mechanism for axial movement of a rod, in particular a plunger rod for an infusion pump. For example the British Patent Specification 2 077 599 discloses an automatic infusion pump to receive an infusion syringe, said pump comprising a battery and a control circuit for a motor driving a gear which drives an axially grooved drive wheel whose axis of rotation is perpendicular to the plunger rod of the infusion syringe. In this known apparatus the plunger rod is pressed against the drive wheel so that the cams of said wheel penetrate into the plunger rod consisting of plastics which is cold deformed by the penetration of the cams.

Provided that the gear has been manufactured with great accuracy, this known apparatus can give a tolerance of about 5 % of the injected amount per unit of time when the unit of time is of the order of several hours. In certain medicaments, e.g. insulin, however, it is desirable to obtain a corresponding metering accuracy even if the mentioned unit of time is only about one hour. Since the patient carries the infusion pump on him, the pump should be as compact as possible, and this entails in practice that the diameter of the known drive wheel is about 5 mm and that an average of four of its about 30 cams or teeth engage the plunger rod. Further, the drive wheel is only to rotate about 1/200 of an entire revolution for the pump to deliver an international unit of insulin, and it will therefore be appreciated that the desired accuracy is difficult to obtain since not only are huge requirements made of the tolerance of the drive wheel tooth cross-section, but also any inhomogeneity in the plunger rod material affects the deformation of the material by each tooth during the entire travel thereof through the material. It will moreover be appreciated that also variations in the effective pitch diameter of the drive wheel because of e.g. eccentricity, bearing wear or impurities in the bottom of the axial grooves of the wheel greatly affect the accuracy of the apparatus.

The object of the invention is to provide a drive mechanism of the above-mentioned type which significantly improves the accuracy over the prior art, while no huge tolerance requirements are made of the gear transmission of the mechanism.

This object is achieved by the use of a worm drive, as stated in the characterizing portion of claim 1, because precisely the use of a worm drive known per se for an apparatus, in which the flanks of one or more worm threads penetrate evenly into the rod material by cold deformation thereof, provides more and surprising advantages than those already known in connection with non-self-cutting worm gears.

First, the relative or short term accuracy is improved since each track formed by the worm threads in the plunger rod has a constant cross-section over a substantial portion of the engagement between the track and the worm, and since more thread flanks than in the prior art engage the rod material, axial flowing of the rod material because of the counter pressure of the plunger rod will be less pronounced. Second, also the absolute or long term accuracy is improved because the transmission does not depend upon a pitch diameter, but only upon the pitch of the worm threads. Finally, it may be stated that the worm mechanism is self-cleaning, and that the large transmission allows the other toothed wheels of the gear transmission to be manufactured with small tolerance requirements and optionally to be plastics moulded.

The friction between the worms and the plunger rod has been found to be relatively great with respect to the energy consumption for the advance of the plunger rod, and particularly in case of mechanisms driven by batteries it is desirable to reduce the frictional energy. This can be obtained by reducing the axial length of the drive worms, whereby, however, the displacement of the plunger rod material will have a relatively great influence on the accuracy, which accordingly varies with the rotation of the drive worms. Since the invention also comprises smoothing of this variation, the characterizing portion of claim 1 sets forth a combination of features providing a significantly greater accuracy with respect to the prior art.

As stated in claim 2, the smoothing of the thread inlet is preferably obtained by mutual angular displacement. To obtain equilibrium for lateral forces, the embodiment defined in claim 3 is preferably used, and this embodiment also entails that the plunger rod is wedged down between the drive worms.

Claim 4 defines alternative features for smoothing of the worm thread inlet so that the metering accuracy is not affected by discontinuous thread inlets.

The thread inlet may be made additionally even by making the drive worms single conical or double conical. The single conical form stated in claim 5 involves a noticeable advantage at the thread inlet, while the double conical form stated in claim 6 also involves smoothing of the thread outlet.

The effect of discontinuous inlets and outlets can be further reduced by the features defined in claim 7, which require the presence of two or more worms.

Claim 8 defines details in a preferred embodiment which is particularly inexpensive to manufacture, in particular for infusion syringes. Especially in connection with the infusion syringe, the invention provides the additional advantage that the counter pressure caused by blocking or by homing of the infusion syringe plunger is transformed to a force which is substantially parallel with the axis of the worm, and which is therefore easy to detect, e.g. by means of the features stated in claim 9.

The invention will be explained more fully by the following description of some embodiments with reference to the drawing, in which:

figs. 1 and 2 show a known, automatic infusion pump,

fig. 3 is an explanatory sketch of the prior art,

figs. 4 and 5 show sections through a preferred embodiment of the mechanism of, the invention,

fig. 6 is a bottom view of the mechanism of figs. 4 and 5, while

figs. 7 - 9 are explanatory sketches of alternative embodiments of the drive mechanism of the invention.

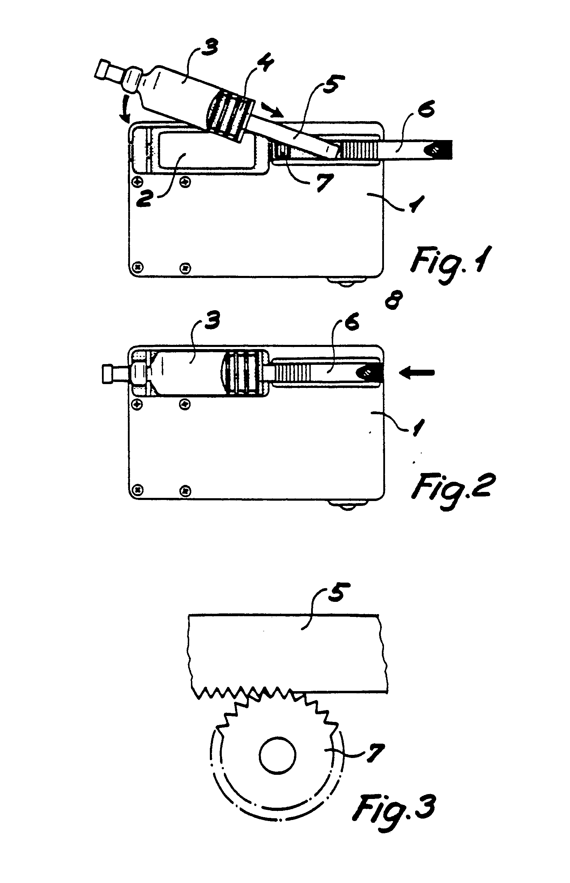

The known structure shown in fig. 1 comprises a housing 1 with a cavity 2 to receive a carpule 3 for an infusion syringe, which moreover comprises a plunger 4 and an associated plunger rod 5 preferably made of plastics. The numeral 6 designates a slide which in the extended position allows the carpule and the plunger rod to be received in the apparatus, following which the slide can be pushed into position, as appears from fig. 2. The slide 6 is arranged to urge the plunger rod 5 down toward a drive roller 7 driven by a motor in the housing 1 via a gear. The housing moreover accomodates electronic means to control the motor so that a specific dosis per unit of time is metered or a predetermined dosis is dispensed each time a switch 8 is activated. Teh apparatus is designed to be carried by a patient, e.g. a diabetic, and the infusion syringe is connected via a plastic hose with a needle inserted into the patient. It is therefore disirable that the apparatus is as compact as possible, and that metering as accurate as possible can be obtained.

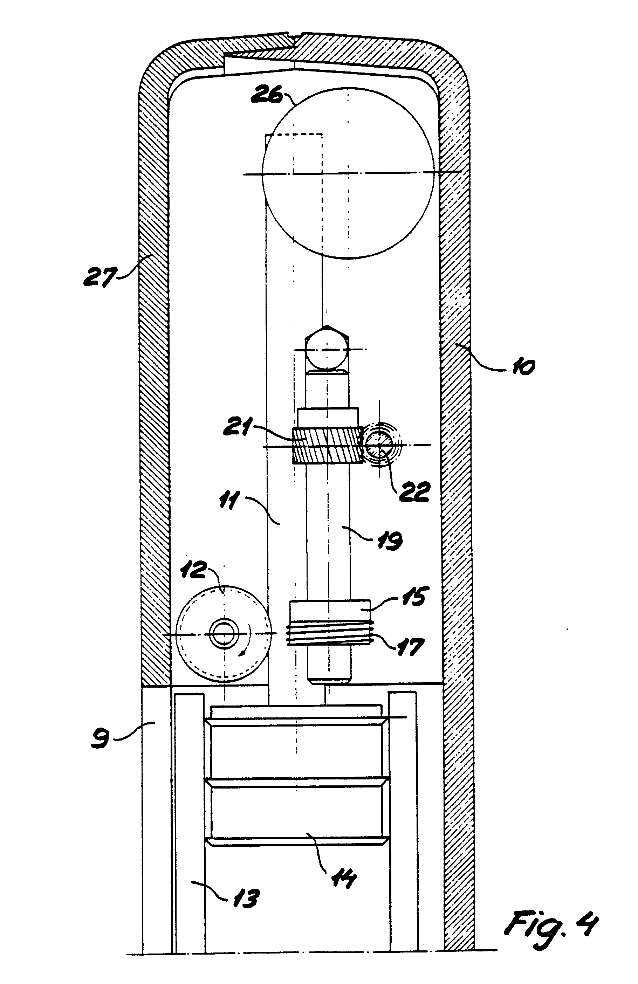

In practice, the drive roller 7 is only about 5 mm in diameter (see fig. 3), and for the apparatus to dispense an international unit of insulin the roller is just to be rotated about 1/200 of an entire revolution. Since only few teeth of the roller 7 are engaged with the plunger rod 5, it is very difficult with this art to obtain an accuracy of about 5 %, based on an insulin unit. There are several sources of inaccuracy, one of them being that each of the drive roller teeth works into the plunger rod 5 by cold deformation of it, said cold deformation continuing over the entire travel of the tooth through the materail. The transmission ratio depends on a pitch diameter, which varies with the function of the depth of tooth penetration into the plunger rod, and it will therefore be appreciated that impurities in the bottom of the teeth, like drive roller bearing wear, affects the accuracy. All these drawbacks are remedied by the mechanism of the invention, a preferred embodiment of whcih is shown in figs. 4 - 6.

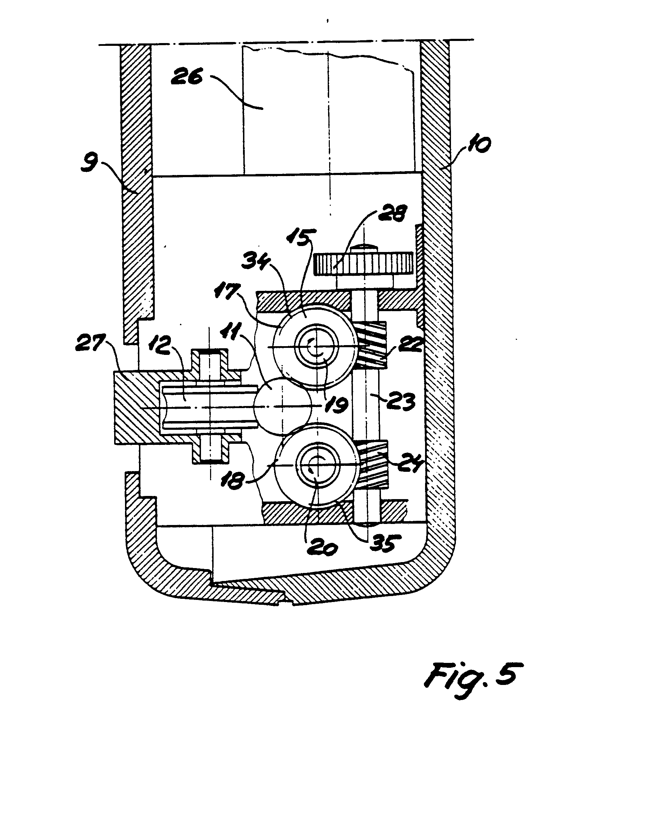

Since the invention concerns the drive engagement with the cold deformable plunger rod alone, only this drive mechanism is shown in figs. 4 - 6, while it will be appreciated that figs. 4 - 6 are taken of an apparatus which might otherwise be constructed as explained in connection with figs. 1 and 2. In fig. 4 the numerals 9 and 10 designate the top side and the underside, respectively, of a housing of the type shown in fig. 1. Fig. 4 shows a section through the housing, a plunger rod 11 being visible which is placed in the apparatus together with the associated plunger 14 and the carpule 13. A roller 12 serves to press the plunger rod 11 down between two worm wheels 15, 16 (fig. 5), which are each provided with one or more threads 17, 18 designed to cut into the relatively soft plunger rod material, which is preferably plastics. Each of the worm wheels 15, 16 is placed on an associated shaft 19, 20 rotated in opposite directions of rotation by means of a common worm or toothed wheel gear. More particularly, the shaft 19 has a helical toothed wheel 21 which is driven by a toothed wheel 22, which is in turn attached to a shaft 23 supporting an additional worm wheel 24 driving the shaft 20. As appears from the arrows in fig. 5, the worms 15, 16 ar driven in opposite directions, thereby preventing the plunger rod 11 from being subjected to a rotational force. The shaft 23 is driven via a toothed wheel 28 via an additional gear transmission by a motot, which is schematically shown at 26.

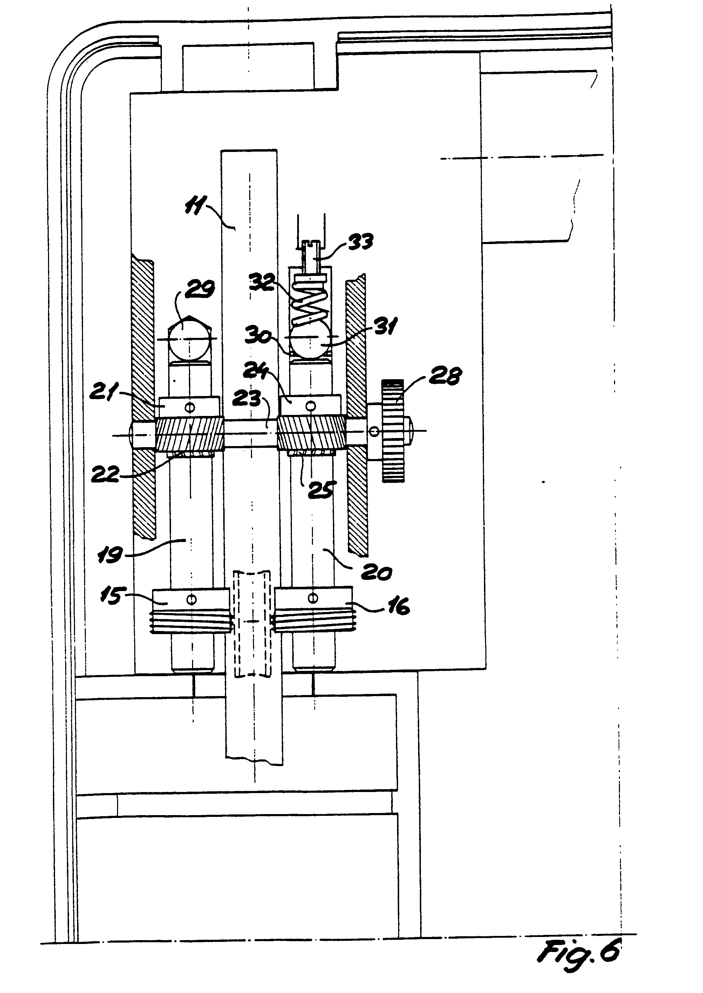

As appears from fig. 5, the backup roller 12 is journalled in a slide 27 (for clarity, this does not appear clearly from fig. 4). The slide 27 serves the same purpose as the slide 6 from fig. 1 so that the plunger rod 11 is kept in place and wedged between the two worm wheels 15, 16. It will thus be appreciated that the plunger rod 11 is driven axially when the shaft 23 rotates, the threads 17, 18 on the worms 15, 16 cutting into the plunger rod 11 while cold deforming it. Fig. 6 is a schematic bottom view of the mechanism of fig. 5, showing clearly the engagement between the wheels nemtioned in connection with fig 5 and the plunger rod 11. Because of the large transmission in the worms, it will be appreciated that the (not shown) toothed wheels in the gear transmission between the toothed wheel 28 and the motor 26 can be made of plastics.

Fig. 6 moreover shows a pair of embodiments of pressure bearings for the shafts 19 and 20, respectively. The shaft 19 can be journalled like the shaft 20, but, in the drawing, it is journalled against a fixed steel ball 29. If the needle should be blocked, or the plunger 14 has bottomed in the carpule 13, a strong counter pressure will be generated in parallel with the shafts 19, 20, which can be detected by means of the pressure bearing embodiment shown in fig. 6 for the shaft 20. The latter pressure bearing comprises a seat rim 30 for the steel ball 31, which is pressed against this seat by means of a spring 32 whose spring force is adjustable by means of a screw 33. Connection of electric leads to the seat rim 30 and the screw 33, respectively, results in the generation of an electric interruption if the shaft 20 is urged to the right by a force exceeding the spring force set by the screw 33. Thus, an alarm for the patient is obtained in a very simple manner, and overloading of the mechanism is avoided.

The most essential advantages of the mechanism of the invention have already been mentioned in the preamble to the description, and they will therefore just be repeated briefly here in relation to the embodiment described above. First, the transmission ratio is very well-defined and depends upon the pitch of the threads on the worms 15, 16, independent of the depth of penetration of the threads into the plunger rod 11. The thread inlet and outlet problems have also been mentioned before, but it has been found in practice that the described embodiment involves a suitable reduction in the action upon the thread inlets in that the thread inlets are mutually angularly displaced, which means that the beginning of the threads (34, 35 in fig. 5) is mutually rotated through an angle of 180°. Preferably, the envelopes of the threads are conical, the vertex being in the direction of the carpule, and the included angle being just a few degrees. The combination of this slightly expanding pitch diameter and the angularly displaced inlets has been found to be particularly advantageous. Corresponding measures may also be taken in connection with the thread outlets, but this is not necessary to obtain the desired accuracy in practice.

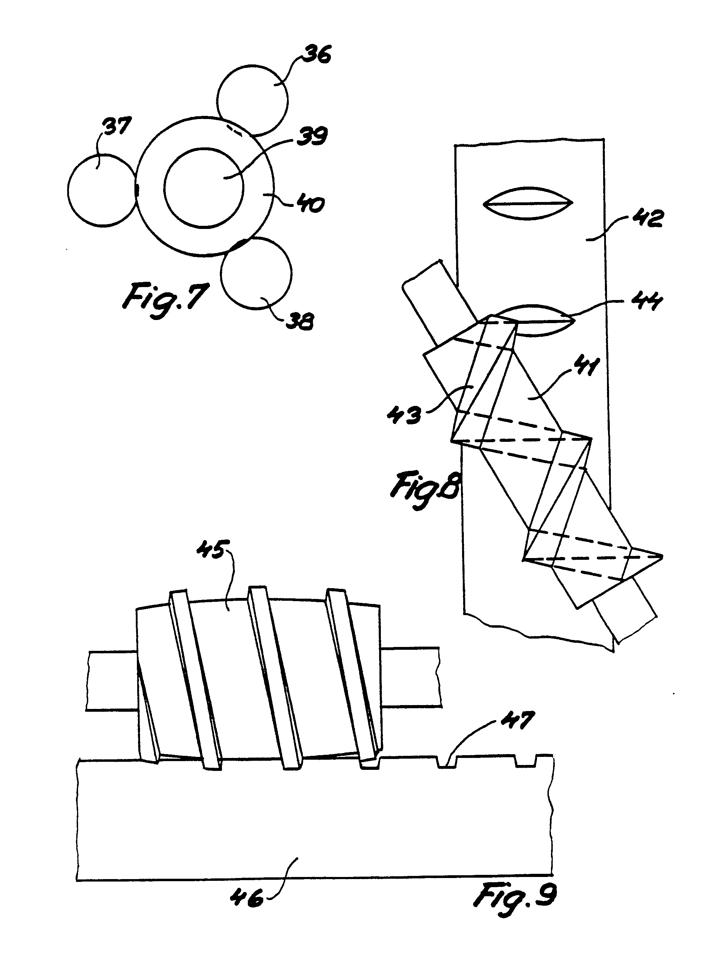

Some alternative embodiments will be described below in connection with figs. 7 - 9. Fig. 7 shows an explanatory sketch of an embodiment comprising three worm wheels 36, 37, 38 designed to drive a plunger rod 39. The plunger rod 39 might be made of a hard material on whose exterior a sleeve 40 of a cold deformable material is placed. Of course, the embodiment shown in fig. 7 is not suitable in connection with the slide described previously, but is useful in a mechanism where the plunger rod is introduced axially with respect to the drive worms 36-38. Provision of several worms results in further smoothing of the inlets, merely by displacing these mutually in an angle of about 120° when three worms are provided.

Fig. 8 is a schematic view of an embodiment in which the axis of rotation of the worm 41 forms an acute angle with the plunger rod 42. The thread 43 is shown with a very great pitch for clarity, and when the axis of rotation of the worm is rotated through the said angle the threads form grooves in the plunger rod 42, said grooves being perpendicular to the plunger rod so that the drive force on the plunger rod 42 is approximately parallel with the direction of movement of the plunger. The numeral 44 designates the grooves produced by the thread 43 in the plunger rod 42, and when the worm 41 extends beyond the area where it cooperates with the plunger rod 42, discoutinuous thread inlet and outlet can be obtained, which is symbolized by the solid lines on the worm, these lines representing the extent of the thread cooperation with the plunger rod 42.

Fig. 9 shows an additional embodiment of a worm mechanism of the invention, where it can be seen that the worm 45 has a barrel-shaped cross-section, which means that the diameter of the threads is greatest at the centre and decreases toward the ends of the worm. The plunger rod is represented at 46, and it will therefore readily be seen that the grooves 47 in the plunger rod are produced continuously as the worm 45 rotates, and the thread emerges continuously and evenly from the grooves 47.

It will be appreciated that the variants shown in figs. 8 and 9 might be incorporated in embodiments of e.g. the type shown in figs. 4 - 6 and 7, thus providing a wide spectrum of optional variations with respect to the desired accuracy and the manufacturing price.

**Claims**

1. A drive mechanism for axial advance of a plunger rod (11, 39, 42, 46), in particular for an injection syringe, said mechanism comprising rotating drive means designed to penetrate into the plunger rod to thereby produce depressions for engagement with projections on the drive means, characterized in that the drive means consist of one or more drive worms (15, 16, 36-38, 41, 45) so shaped that, in operation, a substantially uniform plunger rod and drive means engagement with respect to the rotation of the drive means is produced by the penetration.

2. A drive mechanism according to claim 1, characterized by several drive worms with substantially uniform mutual angular displacement between the respective thread inlets.

3. A drive mechanism according to claim 1 or 2, characterized by two drive worms designed to cooperate with the said material in regions with a mutual arc distance of a out 120°, and by a releasable, idle backup roller (12) designed to press the rod against the drive worms.

4. A drive mechanism according to claims 1 - 3, characterized in that the axis of rotation of the drive worm forms an acute angle with the rod corresponding to the pitch of the worm threads (34, 35), and that the worm threads extend a distance axially beyond said region where the worm threads penetrate into the said material.

5. A drive mechanism according to claims 1 - 4, characterized in that the pitch diameter of the drive worm increases in a direction away from the thread inlet.

6. A drive mechanism according to claim 5, characterized in that the pitch diameter of the drive worm decreases in a direction toward its thread outlet.

7. A drive mechanism according to claims 2 - 6, characterized in that the thread inlet and the thread outlet of the worm are mutually angularly displaced.

8. A drive mechanism according to claim 3 and any of claims 5 - 7, characterized in that the axes of the two drive worms are parallel with the rod, and that the worms are driven in opposite directions of rotation by means of their respective associated toothed wheels, said toothed wheels being driven by a common, additional worm drive (22-24).

9. A drive mechanism according to any of the preceding claims, <u>characterized</u> in that the worm is journalled in a pressure bearing (30-33) containing a pressure detector for axial pressure.

**Patentansprüche**

1. Antriebsvorrichtung zum axialen Vorschub einer Kolbenstange (11, 39, 42, 46), insbesondere für eine Injektionsspritze, mit einem rotierenden Antriebsmittel zum Eindringen in die Kolbenstange, um dadurch Vertiefungen zum Zusammengreifen mit Vorsprüngen an dem Antriebsmittel zu schaffen, <u>dadurch gekennzeichnet, daß das</u> Antriebsmittel aus einer oder mehreren Antriebsschnecken (15, 16, 36-38, 41, 45) besteht, die so geformt sind, daß bei Betrieb durch das Eindringen ein im wesentlichen gleichförmiges Zusammengreifen von Kolbenstange und Antriebsmittel in Bezug auf die Rotation des Antriebsmittels erfolgt.

2. Antriebsvorrichtung nach Anspruch 1, gekennzeichnet durch mehrere Antriebsschnecken mit im wesentlichen gleichförmiger gegenseitiger Winkelverlagerung zwischen den jeweiligen Gewindeeinlässen.

3. Antriebsvorrichtung nach Anspruch 1 oder 2, gekennzeichnet durch zwei Antriebsschnecken, die mit dem Material in Bereichen mit einem gegenseitigen Bogenabstand von etwa 120° zusammenwirken, und durch eine lösbare, leerlaufende Stützrolle (12) zum Drücken der Stange gegen die Antriebsschnecken.

4. Antriebsvorrichtung nach den Ansprüchen 1 - 3, dadurch gekennzeichnet, daß die Rotationsachse der Antriebsschnecken einen spitzen Winkel mit der Stange bildet, welcher der Steigung der Schneckengewinde (34, 35) entspricht, und daß sich die Schneckengewinde um einen Abstand axial über den Bereich hinaus erstrecken, in dem die Schneckengewinde in das Material eindringen.

5. Antriebsvorrichtung nach den Ansprüchen 1 - 4, dadurch gekennzeichnet, daß der Flankendurchmesser der Antriebsschnecke in der vom Gewindeeinlaß wegweisenden Richtung zunimmt.

6. Antriebsvorrichtung nach Anspruch 5, dadurch gekennzeichnet, daß der Flankendurchmesser der Antriebsschnecke in der zum Gewindeauslaß der Antriebsschnecke weisenden Richtung abnimmt.

7. Antriebsvorrichtung nach den Ansprüchen 2 - 6, dadurch gekennzeichnet, daß der Gewindeeinlaß und der Gewindeauslaß der Schnecke zueinander unter einem Winkel versetzt sind.

8. Antriebsvorrichtung nach Anspruch 3 und einem der Ansprüche 5 - 7, dadurch gekennzeichnet, daß die Achsen der beiden Antriebsschnecken parallel zu der Stange verlaufen, und daß die Schnecken mittels ihrer jeweiligen zugeordneten Zahnräder in entgegengesetzten Rotationsrichtungen angetrieben sind, wobei die Zahnräder von einem gemeinsamen zusätzlichen Schneckenantrieb (22-24) angetrieben sind.

9. Antriebsvorrichtung nach einem der vorhergehenden Ansprüche, dadurch gekennzeichnet, daß die Schnecke in einem Drucklager (30-33) gelagert ist, welches einen Druckdetektor für Axialdruck aufweist.

**Revendications**

1. Mécanisme d'entraînement pour l'avance axiale d'une tige de plongeur (11, 39, 42, 46), en particulier pour une seringue d'injection, ledit mécanisme comprenant des moyens d'entraînement en rotation conçus pour pénétrer dans la tige du plongeur afin de produire des creux pour une entrée en prise avec des saillies sur les moyens d'entraînement, caractérisé en ce que les moyens d'entraînement comprennent une ou plusieurs vis sans fin d'entraînement (15, 16, 36-38, 41, 45) configurées de manière que, en fonctionnement, la pénétration produise une entrée en prise sensiblement uniforme entre la tige du plonqeur et les moyens d'entraînement par rapport à la rotation des moyens d'entraînement.

2. Mécanisme d'entraînement selon la revendication 1, caractérisé par plusieurs vis sans fin d'entraînement dont les entrées des filets respectives sont décalées mutuellement d'angles sensiblement uniformes.

3. Mécanisme d'entraînement selon la revendication 1 ou 2, caractérise par deux vis sans fin d'entraînement conçues pour coopérer avec ladite matière dans des zones écartées mutuellement d'un arc d'environ 120°, et par un galet fou et libérable (12) d'appui conçu pour presser la tige contre les vis sans fin d'entraînement.

4. Mécanisme d'entraînement selon les revendications 1 - 3, caractérisé en ce que l'axe de rotation des vis sans fin d'entraînement forme un angle aigu avec la tige, correspondant au pas des filets (34, 35) des vis sans fin, et en ce que les filets des vis sans fin s'étendent axialement sur une distance allant au-delà de ladite zone où les filets de la vis sans fin pénètrent dans ladite matière.

5. Mécanisme d'entraînement selon les revendications 1 - 4, caractérisé en ce que le diamètre primitif de la vis sans fin d'entraînement augmente dans une direction s'éloignant de l'entrée des filets.

6. Mécanisme d'entraînement selon la revendication 5, caractérisé en ce que le diamètre primitif de la vis sans fin d'entraînement diminue en direction de sa sortie des filets.

7. Mécanisme d'entraînement selon les revendications 2 - 6, caractérisé en ce que l'entrée de filets et la sortie de filets de la vis sans fin sont décalées angulairement l'une par rapport à l'autre.

8. Mécanisme d'entraînement selon la revendication 3 et l'une quelconque des revendications 5 - 7, caractérisé en ce que les axes des deux vis sans fin d'entraînement sont parallèles à la tige, et en ce que les vis sans fin sont entraînées dans des sens opposés de rotation au moyen de leurs roues dentées associées respectives, lesdites roues dentées étant entraînées par un entraînement supplémentaire commun (22-24) à vis sans fin.

9. Mécanisme d'entraînement selon l'une quelconque des revendications précédentes, caractérisé en ce que la vis sans fin tourillonne dans un palier de pression (30-33) contenant un détecteur de pression axiale.

Fig.1

Fig.2

Fig.3

Fig. 4

Fig. 5

Fig.6

Fig.7

Fig.8

Fig.9

36

37

39

40

38

42

44

41

43

45

47

46